# EUROPÄISCHE PATENTSCHRIFT

(11) **EP 1 661 972 B1**
(45) Veröffentlichungstag und Bekanntmachung des Hinweises auf die Patenterteilung: **17.10.2018**
(21) Anmeldenummer: 05405675.9
(22) Anmeldetag: 29.11.2005
(51) Int. Cl.: A23D 9/06, A61K 35/60, C11B 1/06, C11B 1/10, C11B 5/00, C11B 13/00, A23L 33/115

(54) **Verfahren zur Extraktion von lipohilen Anteilen aus wasserhaltigen Substraten**
Method for extracting lipophilic elements from aqueous substrates
Procédé d'extraction d'entités lipophiles de substrats aqueux

(30) Priorität: 30.11.2004 CH 19702004
(43) Veröffentlichungstag der Anmeldung: 31.05.2006
(73) Patentinhaber: Alpinamed AG, 9306 Freidorf (CH)
(72) Erfinder: Häcker, Peter, 9320 Arbon (CH); Wipfler, Andreas, 9315 Neukirch (Egnach)TG (CH)
(74) Vertreter: Frei Patent Attorneys

(56) Entgegenhaltungen:
- EP-A- 1 378 243
- WO-A1-00/78903
- US-A- 4 716 218
- M. MIKI ET AL.: "Extraction of fish oil from tuna flesh" BULLETIN OF THE FACULTY OF FISHERIES HOKKAIDO UNIVERSITY, Bd. 28, Nr. 4, 1977, Seiten 202-206, XP008045327
- DATABASE WPI Section Ch, Week 199740 Derwent Publications Ltd., London, GB; Class B05, AN 1997-431382 XP002323444 -& JP 09 194362 A (BIZEN KASEI KK) 29. Juli 1997 (1997-07-29)
- FRANKEL E N ET AL: "EVALUATION OF ANTIOXIDANT ACTIVITY OF ROSEMARY EXTRACTS, CARNOSOL AND CARNOSIC ACID IN BULK VEGETABLE OILS AND FISH OIL AND THEIR EMULSIONS" JOURNAL OF THE SCIENCE OF FOOD AND AGRICULTURE, ELSEVIER APPLIED SCIENCE PUBLISHERS. BARKING, GB, Bd. 72, Nr. 2, 1. Oktober 1996 (1996-10-01), Seiten 201-208, XP000626755 ISSN: 0022-5142
- ISABEL MEDINA ET AL.: "Activity of plant extracts for preserving functional food containing n-3-PUFA" EUROPEAN FOOD RESEARCH AND TECHNOLOGY, Bd. 217, Nr. 4, 2003, Seiten 301-307, XP008045278 DESPRINGER VERLAG, HEIDELBERG
- DATABASE WPI Section Ch, Week 200475 Derwent Publications Ltd., London, GB; Class B04, AN 2004-764263 XP002323445 & NZ 510 407 A (HEALTHERIES NEW ZEALAND LTD) 28. Mai 2004 (2004-05-28)
- DATABASE WPI Section Ch, Week 199601 Derwent Publications Ltd., London, GB; Class B04, AN 1996-006899 XP002323446 -& JP 07 285873 A (POLA CHEM IND INC) 31. Oktober 1995 (1995-10-31)
- DATABASE WPI Section Ch, Week 200125 Derwent Publications Ltd., London, GB; Class B04, AN 2001-242983 XP002323447 & RU 2 162 647 C2 (POLAR SEA FISHING OCEANOGRAPHY RES INST) 10. Februar 2001 (2001-02-10)

## Beschreibung

Es ist bekannt, wie man bspw. die Omega-3 Fettsäuren aus tierischem Gewebe wie Fisch, Muscheln, Krustazeen etc. möglichst schonend extrahiert. Das Problem bei solchen Extraktionen ist, dies unter Ausschluss allfälliger Oxidation zu bewerkstelligen und einen möglichst niedrigen Wassergehalt im Extrakt oder den Edukten zu erhalten. Es ist beispielsweise aus US 6,083,536 oder US 6,346,278 bekannt, Omega-3 Fettsäuren aus bspw. Muschelfleisch mittels der sogenannten Kohlensäureextraktion zu gewinnen. Dies wird bewerkstelligt mit superkritischer Kohlensäure. Dies ist ein Mittelding aus Gas und Flüssigkeit. Superkritisches Kohlendioxid ist genauso dicht wie eine Flüssigkeit, hat aber dieselbe Viskosität, denselben Fließwiderstand wie ein Gas. Erreicht wird dieser superkritische Zustand schon bei einer Temperatur von 32 Grad. Allerdings muss man das Kohlendioxid dabei unter Druck setzen, muss es in einem Druckgefäß auf 73 Atmosphären zusammenpressen. Technisch ist das kein Problem.

Das Besondere: Superkritische Kohlensäure ist dank ihrer Fließfähigkeit eine überaus agile Substanz. Dadurch kann sie sich in andere Stoffe bestens hineindrängen und sie auflösen. Auf diese Weise kann superkritische Kohlensäure manch organisches Lösemittel ersetzen und vermag bspw. auch Kaffee zu entkoffeinieren.

Dieser Prozess hat Nachteile. Einerseits ist er technisch und verfahrensmässig aufwendig, andererseits ist es schwierig mit dem relativ hohen wässrigen Anteil umzugehen. Ferner ist Kohlendioxid nicht das geeignete Schutzmittel gegen Oxidation, da Spuren von freiem Sauerstoff nicht ausgeschlossen werden können.

Eine weitere bekannte Technik ist das Gefriertrocknen. Dabei wird das tierische Gewebe gefriergetrocknet und somit alles Wasser bis auf einen Restgehalt entfernt. Aus dem Trockensubstrat werden die lipoiden Anteile mit einem lipophilen Lösungsmittel eluiert. Diese Lösungsmittel sind Aether, niedrig siedende Petrolaether, Extraktionsbenzin bzw. Hexan und ähnliche Eluiermittel, die nach dem Verdampfen das Konzentrat hergeben. Auch dieser Prozess hat Nachteile. Ausser dem Gefriertrocknen sind alle weiteren Prozessschritte nicht mehr 'lebensmitteltechnisch', sondern in einem gewissen Sinne ,chemisch'. Nicht eliminierbare Rückstände der Lösungsmittel sind unerwünscht, ebenso chemisch nicht eliminierbare Rückstände der Lösungsmittel. Die Dämpfe bilden gemeinsam mit Luft gefährliche, explosive Gemische und verlangen zudem auch aus toxikologischer Sicht erhöhten Arbeitsschutz.

M. Miki et al. offenbaren im Bulletin of the Faculty of Fisheries Hokkaida University, Bd. 28, Nr.4 1977 ein Verfahren zur Extraktion von Fischöl aus Thunfisch mit verschiedenen Lösungsmitteln, um die Extraktionsrate zu optimieren.

In WO 00/78903 wird eine Extraktion von Lipiden aus frischen biologischem Gewebe offenbart, welches die Ausbeute optimieren soll, durch Hacken des biologischen Materials in der Gegenwart von Säure und anschliessender oder gleichzeitiger Extraktion mit einem Lösungsmittel.

Es ist eine Aufgabe der Erfindung, ein spezielles Verfahren zu finden, mittels welchem aus einem wasserhaltigen Substrat mit lipoiden Anteilen, diese quantitativ zu extrahieren, das heisst, vom Wasseranteil zu befreien oder den freien Wasseranteil zu binden.

Eine weitere Aufgabe der Erfindung ist das Verhindern hydrolytischer Prozesse in Gegenwart von Enzymen, mögliche oxidative Einwirkung in jeder Produktionsphase, sowie im Prozess als auch am Endprodukt, auszuschliessen, sei es von externer Seite oder im Lösungsvorgang selbst.

Dies wird durch das erfindungsgemässe Verfahren gemäss den Patentansprüchen erreicht.

### DARSTELLUNG DER ERFINDUNG

Das erfindungsgemässe Verfahren wird nun am Beispiel eines überaus heiklen Stoffes zur Extraktion diskutiert. Natürlich kann das Verfahren auch auf weniger heikle Materialien angewendet werden, wo schonende Extraktion gewünscht wird.

Das Muschelfleisch der Grünlippmuschel (Perna Canaliculus) enthält nebst weiteren klinisch wirksamen zum Beispiel antiinflammatorischen Komponenten wie bspw. Glycosaminglykane auch einen erheblichen Anteil an Omega-3 Fettsäuren wie beispielsweise Eicosapentaensäure (EPS bzw. EPA a für acid) und/oder Docosahexaensäure (DHS bzw. DHA), wie sie besonders in fettigen Fischen vorkommen. Der Grünlippmuschel wird eine Wirksamkeit bspw. bei arthritischen Beschwerden attestiert, so wird das Extrakt aus der Grünlippmuschel, sofern es möglichst unverändert in eine galenische Fertigungsform überführt werden kann, diese günstige Wirkung beibehalten.

EPA und DHA sind Omega-3-Fettsäuren, die strukturell anders sind als die Omega-6-Fettsäuren, die in Pflanzen und Tieren nicht aquatischen Ursprungs zu finden sind. Sie haben eine grössere Anzahl von Doppelbindungen, die sie empfindlicher für Oxidierung macht. Dadurch weisen sie eine höhere Neigung zum Polymerisieren, zum Zyklieren oder zur Stereomutation der Doppelbindungen durch Sauerstoffeinfluss auf.

Azeotropes Aethylalkohol-Wassergemisch siedet bei 78.15° C (reiner Alkohol bei 78.3° C) und enthält 4% Wasser. Aethylalkohol, kurz Alkohol, ist in geringen Mengen für den Menschen unschädlich. Man findet ihn in vielen Lebensmitteln, bspw. Konfitüren durch unvermeidbare Gärung des Zuckers, und hat somit als Rückstand im Produkt keine nachteilige Wirkung. Nebst günstigen Eigenschaften als Solvent ist dies mit ein Grund, als Extraktionsmittel bevorzugt Aethylalkohol zu verwenden. Da auch höhere Alkohole in Lebensmitteln vorkommen, bspw. beim Wein, eignen sich auch diese, wenn sie bevorzugt azeotrope Charakteristika aufweisen. Andererseits ist es auch ein Grund für die Niedrighaltung der Temperatur beim Abdestillieren des Lösungsmittels mit C₂H₅OH zu arbeiten: Mit jedem zusätzlichen C-Atom in einem Alkohol steigt der Siedepunkt um ungefähr 19° C an.

Das Muschelfleisch (frisch oder tiefgefroren) enthält ca. 60 - 70 % Wasser. Eine bestimmte Menge von Muschelfleisch und zwar grundsätzlich (tiefgefrorenes) Frischfleisch wird mit 96% Alkohol versetzt und zwar soviel, dass der Alkohol durch den Wassergehalt im Muschelfleisch auf ungefähr 75% verdünnt wird und damit immer noch genügend hochprozentig ist, um die Extrahierwirkung für lipoide Stoffe beizubehalten. Anschliessend wird das Muschelfleisch gut abgepresst und der Presskuchen mit soviel absolutem, also 100 % Alkohol extrahiert, dass der Wassergehalt im Alkohol 10% nicht übersteigt. Die Entfernung des Restwassers mit 100% Alkohol ist unter anderem auch darum vorgesehen, dass keinerlei Trocknungsmittel wie bspw. Na-Sulfat verwendet werden muss, welche unter Umständen ein Reaktionspotential mit Phosphatiden bzw. Phospholipiden im Konzentrat bilden können.

Diese beiden Ansätze werden alsdann vereinigt und das Alkohol-Wassergemisch schonend abdestilliert.

Dies ist ein sehr heikler Vorgang. Es hat sich gezeigt, dass praktisch alle Destillationsmethoden das zu gewinnende Konzentrat in einem gewissen Sinne schädigen, die angezielte Wirkung herabstufen bzw. zu einem Produkt führen, dem man nicht die gewünschte Qualitätsstufe zugestehen kann.

Zum Entfernen des Alkohol-Wassergemisches wurde mit hoch befriedigendem Resultat die Dünnschichtzentrifugalverdampfung verwendet. Als Schutzgas ist Stickstoff ausreichend. Allenfalls kann man dem Stickstoff geringe Mengen einer reduzierenden Komponente beifügen, um unerwünschte Redoxreaktionen im Konzentrat zu vermeiden.

Das gewonnene Konzentrat enthält maximal 0.5 %Alkohol. Dies alles geschieht bei ca. 50°C.

Gemäss diesem Verfahren ist der Gehalt von sehr leicht zersetzbaren Phosphatiden bzw. Phospholipiden besonders hoch. Es wurden Werte bis zu 40 % Anteil im Konzentrat erzielt, hohe Werte wie 20% bis 50%, vorzugsweise 20-40%, z. B. 30%-40%, sind in der Regel im Konzentrat erzielbar. Vorzugsweise enthält das Konzentrat einen Anteil von mindestens 20% an Phospholipiden. Der markant hohe Gehalt an Phospholipiden, sowie vorzugsweise auch zusätzlich von antiinflammatorisch wirkenden Zoosterolen ist ein Charakteristikum des Fertigproduktes. Solche Zoosterole sind insbesondere weitere Zoosterole, ausser dem aus dem Fischöl bekannten Cholesterol, wie beispielsweise Campesterol, Stigmasterol, Betasitosterin etc.. Die Werte dieser Zoosterole bewegen sich vorzugsweise in einem minimalen Bereich von 0.1-0.8%, typischerweise von 0.2-0.7%, z.B. 0.5 %.

Muschelfleisch enthält wie Fischfleisch Stickstoffverbindungen, die bei Zersetzung bspw. durch Methylamine wie Trimethylamine etc. den unangenehmen Fischgeruch hervorrufen. Die Zersetzung kann durch enzymatische Prozesse hervorgerufen werden oder aber durch Oxydation. Den zersetzerischen Enzymeinfluss kann man z. B. durch Kalthalten des Muschelfleisches, wie beispielsweise durch tiefgefrieren oder Deaktivieren der Enzyme durch Kurzzeiterhitzung, vermeiden. Bei der oben diskutierten Extraktion spielen die Enzyme keine Rolle mehr, da sie im hochprozentigen alkoholischen Milieu keine für sie günstige Bedingungen finden; sie werden darin denaturiert. Jedoch ist der Einfluss des Sauerstoffs abzuwehren. Trotz Schutzgas schädigt der im Extraktionsgut und im Extrahiermittel gelöste Sauerstoff, auch wenn es geringe Mengen sind, die relativ wenig stabilen Stickstoffverbindungen, weswegen ein Antioxidans schon bei der Extraktion beigegeben wird, bspw. dem Alkohol zugesetzt. Sehr bewährt hat sich die Verwendung von Camosolsäure, welche durch die Beigabe von Rosmarinblättern daraus, während dem Herstellprozess extrahiert wird. Carnosolsäure ist ein sehr wirksames Antioxidans (Radikalfänger).

Extrahiert man Grünlippmuschelfleisch, so erhält man eine relativ geringe Menge Lipide im Verhältnis von ca. 40 : 1 (frisches Grünlippmuschelfleisch : nativem Extrakt). Es ist üblich, der Lipid-Fraktion von Grünlippmuschelfleisch einen geeigneten Träger, im vorliegenden Falle Fischöl, welches ebenfalls die Omega-3-Fettsäuren enthält, beizumischen. Dabei hat es sich gezeigt, dass das im Konzentrat enthaltene Antioxidans mit entsprechendem Ueberschuss auch im Fischöl die Methylaminbildung sowie sonstige Oxidationen verhindert.

### Herstellung von 1000 g Endprodukt (Nahrungsergänzungspräparat):

3600 - 4000 g Muschelfleisch tiefgefroren
7200 - 8000 g 96% Alkohol
18 -20 g Rosmarinblätter (Antioxidans enthaltend Carnosolsäure)

Das Muschelfleisch wird im extrahierenden und gleichzeitig schützenden Alkohol unter Zusatz von Rosmarin zerkleinert (gemixt) und anschliessend abgepresst und filtriert. Es resultiert ein erster Ansatz von lipoidem Material in ca. 75% Alkohol und ein Pressrückstand von zerkleinertem Muschelfleisch, der seinerseits noch zu gewinnendes lipoides Material enthält.
ca. 2000 g Pressrückstand
ca. 4500 - 5000 g Alkohol 100%
18 -20 g Rosmarinblätter

Der Pressrückstand aus zerkleinertem und vorextrahiertem Muschelfleisch wird mit dem 100 prozentigen Alkohol extrahiert unter Zusatz von Rosmarinblätter, anschliessend abgepresst und filtriert. Es resultieren ein zweiter Ansatz von lipoidem Material in ca. 92% Alkohol und der übrigbleibende Pressrückstand, der nun verworfen werden kann.

Das Alkohol-Wassergemisch der beiden Ansätze 1 und 2 wird unter Schutzgas vorsichtig in einem Dünnschichtzentrifugalverdampfer unter Vakuum bei max. 50°C abdestilliert. Der Rückstand, die gewonnene Lipid-Fraktion, beträgt ca. 100 Gramm und wird ständig unter Luftabschluss gehalten.

Selbstverständlich kann dieses Verfahren auch für die Extraktion von lipoiden Substanzen aus Fischfleisch, aus Krustaceen, aus Organen wie Leber, Niere, Gehirn, Pankreas, aus Muskelfleisch, wie gesagt aus wasserhaltigen Substraten dieser Gattung angewendet werden.

**Zur Herstellung des Nahrungsergänzungspräparats** werden 100 g der Lipidfraktion mit 900 g reinem Fischöl vermischt. In der nachträglichen Analyse soll das Antioxidans, wie im vorliegenden Falle Carnosolsäure in einer geeigneten Konzentration, im Fertigprodukt noch nachweisbar sein bspw. mittels HPLC (high performance liquid chromatography). Damit ist gesichert, dass alle Zwischenprodukte und schliesslich auch das Endprodukt gegen Oxidation geschützt sind. Ferner soll das Endprodukt einen Mindestgehalt zwischen 20% bis 40 % an Phospholipiden, sowie vorzugsweise erhebliche Mengen, z. B. im Bereich von ca. 0.5%, von antiinflammatorisch wirkenden Zoosterolen. aufweisen.

Das Endprodukt kann dann in die gewünschte galenische Form gebracht werden, vorzugsweise Softgel-Kapseln, um den Sauerstoffkontakt bei der Lagerung auszuschliessen. Die ursprünglichen Enzyme sind entweder inaktiviert oder so denaturiert, dass sie das Produkt nicht mehr verändern. Man kann davon ausgehen, dass durch das erfinderische Verfahren ein stabiles Produkt aus einem überaus heiklen Ausgangsmaterial hergestellt werden kann.

Mit anderen Worten: Bei dem erfindungsgemässe Verfahren zur Extraktion lipoider Anteile enthaltend weitere Zoosterole ausser Cholesterol aus wasserhaltigem tierischem frischem oder tiefgefrorenem Gewebe wird das frische oder tiefgefrorene Gewebe in einer ersten Stufe mit einem mit Wasser azeotropen 96% Ethylalkohol, extrahiert, wobei soviel des 96% Ethylalkohols zugesetzt wird, so dass der Alkoholgehalt in einem ersten Filtrat mindestens ungefähr 75% beträgt. In einem zweiten Schritt wird das Extrakt abgepresst und filtriert und der Rückstand mit einem 100% Ethylalkohol ausgewaschen und filtriert, wobei soviel des 100% Alkohols dem Rückstand zugesetzt wird, dass der Wassergehalt im zweiten Filtrat nicht mehr als 10% beträgt. Anschliessend werden die beiden Filtrate vereinigt und das Alkohol/Wassergemisch wird abdestilliert.

## Patentansprüche

1. Verfahren zur Extraktion lipoider Anteile enthaltend weitere Zoosterole ausser Cholesterol aus wasserhaltigem tierischem frischem oder tiefgefrorenem Gewebe, **dadurch gekennzeichnet,**
**dass** das frische oder tiefgefrorene Gewebe in einer ersten Stufe mit einem mit Wasser azeotropen 96% Ethylalkohol, extrahiert wird, wobei soviel des 96% Ethylalkohols zugesetzt wird, so dass der Alkoholgehalt in einem ersten Filtrat mindestens ungefähr 75% beträgt,
**dass** das Extrakt abgepresst und filtriert und der Rückstand mit einem 100% Ethylalkohol ausgewaschen und filtriert wird, wobei soviel des 100% Alkohols dem Rückstand zugesetzt wird, dass der Wassergehalt im zweiten Filtrat nicht mehr als 10% beträgt, und dass die beiden Filtrate vereinigt und das Alkohol/Wassergemisch abdestilliert wird.

2. Verfahren gemäss Anspruch 1, **dadurch gekennzeichnet, dass** ein Antioxidans der ersten Extraktion zugesetzt wird.

3. Verfahren gemäss einem der Ansprüche 1-2, **dadurch gekennzeichnet, dass** das Alkohol/Wassergemisch ohne Verwendung eines Trocknungsmittels mittels eines Dünnschichtzentrifugalverdampfers bei gleichzeitiger Stickstoffbegasung abdestilliert wird.

4. Verfahren gemäss einem der Ansprüche 1 bis 3, **dadurch gekennzeichnet, dass** Meeresfrüchte und/oder Fisch zur Extraktion der lipoiden Anteile eingesetzt wird.

5. Verfahren gemäss Anspruch 2, **dadurch gekennzeichnet, dass** dem Ethylalkohol und jeder Zwischenstufe bis zu einem Endprodukt ein geeignetes Antioxidans zugesetzt wird.

6. Verfahren nach Anspruch 4, **dadurch gekennzeichnet, dass** die Menge des Antioxidans so bemessen wird, dass im gewonnenen, fertigen Endprodukt eine Restmenge vorhanden bleibt, die einen bleibenden Oxidationsschutz darstellt.

7. Verfahren nach einem der Ansprüche 2, 5 oder 6, **dadurch gekennzeichnet, dass** als Antioxidans Carnosolsäure, in Form von Rosmarin zugesetzt wird.

8. Produkt erhältlich aus einem Verfahren nach einem der Ansprüche 1-7 mit mindesten 20%, vorzugsweise zwischen 30 % und 40 % Phospholipide, **dadurch gekennzeichnet, dass** es mindestens 0.5% weitere Zoosterole, mit Ausnahme von Cholesterol, aufweist.

9. Produkt nach Anspruch 8, **dadurch gekennzeichnet, dass** es antioxidierende Mittel enthält.

10. Produkt nach Anspruch 9, **dadurch gekennzeichnet, dass** das antioxidierende Mittel Carnosolsäure ist.

## Claims

1. A method for the extraction of lipoidal shares comprising further zoosterols except for cholesterol, from aqueous, animal, fresh or deep-frozen tissue, **characterised in that**
the fresh or deep-frozen tissue is extracted in a first stage with a 96% ethyl alcohol which is azeotropic with water, wherein so much of the 96% ethyl alcohol is added, that the alcohol content in a first filtrate is at least roughly 75%,
that the extract is pressed off and filtered and the residue is washed out with a 100% ethyl alcohol and filtered, wherein so much of the 100% alcohol is added to the residue that the water content in the second filtrate is not more than 10%, and that both filtrates are unified and the alcohol/water mixture is distilled off.

2. A method according to claim 1, **characterised in that** an antioxidant is added to the first extraction.

3. A method according to one of the claims 1-2, **characterised in that** the alcohol/water mixture is distilled off without the use of a drying agent, by way of a centrifugal thin-film evaporator given a simultaneous gassing with nitrogen.

4. A method according to one of the claims 1 to 3, **characterised in that** seafood and/or fish is used for the extraction of the lipoidal shares.

5. A method according to claim 2, **characterised in that** a suitable antioxidant is added to the ethyl alcohol and to each intermediate stage up until the end product.

6. A method according to claim 4, **characterised in that** the quantity of antioxidant is measured such that a residual quantity which represents a lasting oxidation protection remains present in the obtained, finished end-product.

7. A method according to one of the claims 2, 5 or 6, **characterised in that** carnosic acid in the form of rosemary is added as an antioxidant.

8. A product obtainable from a method according to one of the claims 1 to 7 with at least 20%, preferably between 30 % and 40 % phospholipids, **characterised in that** it comprises at least 0.5 % further zoosterols, with the exception of cholesterol.

9. A product according to claim 8, **characterised in that** it comprises antioxidant.

10. A product according to claim 9, **characterised in that** the antioxidant is carnosic acid.

## Revendications

1. Procédé d'extraction de fractions lipoïdes contenant d'autres zoostérols que le cholestérol à partir d'un tissu animal frais ou congelé contenant de l'eau, **caractérisé en ce que**
le tissu frais ou congelé est extrait lors d'une première étape avec un alcool éthylique à 96 % formant un azéotrope avec l'eau, suffisamment de l'alcool éthylique à 96 % étant ajouté pour que la teneur en alcool dans un premier filtrat soit d'au moins environ 75 %,
l'extrait est comprimé et filtré, et le résidu est lavé avec un alcool éthylique à 100 % et filtré, suffisamment de l'alcool à 100 % étant ajouté au résidu pour que la teneur en eau dans le deuxième filtrat ne soit pas supérieure à 10 %, et les deux filtrats sont réunis et le mélange alcool/eau est distillé.

2. Procédé selon la revendication 1, **caractérisé en ce qu'**un antioxydant est ajouté à la première extraction.

3. Procédé selon l'une quelconque des revendications 1 et 2, **caractérisé en ce que** le mélange alcool/eau est distillé sans utiliser d'agent siccatif au moyen d'un évaporateur centrifuge à couche mince avec gazage d'azote simultané.

4. Procédé selon l'une quelconque des revendications 1 à 3, **caractérisé en ce qu'**un fruit de mer et/ou un poisson sont utilisés pour l'extraction des fractions lipoïdes.

5. Procédé selon la revendication 2, **caractérisé en ce qu'**un antioxydant approprié est ajouté à l'alcool éthylique et à chaque intermédiaire jusqu'à un produit final.

6. Procédé selon la revendication 4, **caractérisé en ce que** la quantité de l'antioxydant est déterminée de telle sorte qu'une quantité résiduelle reste dans le produit final fini obtenu, qui constitue une protection contre l'oxydation restante.

7. Procédé selon l'une quelconque des revendications 2, 5 ou 6, **caractérisé en ce que** de l'acide carnosique est ajouté sous la forme de romarin en tant qu'antioxydant.

8. Produit, pouvant être obtenu par un procédé selon l'une quelconque des revendications 1 à 7, contenant au moins 20 %, de préférence entre 30 % et 40 % de phospholipides, **caractérisé en ce qu'**il comprend au moins 0,5 % d'autres zoostérols que le cholestérol.

9. Produit selon la revendication 8, **caractérisé en ce qu'**il contient des agents antioxydants.

10. Produit selon la revendication 9, **caractérisé en ce que** l'agent antioxydant est l'acide carnosique.
